# EUROPEAN PATENT SPECIFICATION

(11) **EP 0 365 556 B1**
(45) Date of publication and mention of the grant of the patent: **12.07.1995**
(21) Application number: 88905320.3
(22) Date of filing: 26.05.1988
(51) Int. Cl.: C07H 19/06, A61K 31/70

(54) **NUCLEOSIDE ANALOGUES**
NUCLEOSID-ANALOGE
ANALOGUES DE NUCLEOSIDES

(30) Priority: 05.06.1987 US 58304; 04.05.1988 US 190273
(43) Date of publication of application: 02.05.1990
(73) Proprietor: GENENTECH, INC., South San Francisco, CA 94080 (US)
(72) Inventor: WEBB, Thomas, R., Belmont, CA 94002 (US)
(74) Representative: Armitage, Ian Michael
(86) International application number: US8801812
(87) International publication number: WO8809796

(56) References cited:
- Chemical Abstracts, vol. 87, 1977 (Columbus, Ohio, US) Ueda, Tohru et al.: "Nucleosides and nucleotides. XV. Synthesis of 2',3'- episulfides derived from uridine" see page 580
- Chemical Abstracts, vol. 87, 1977, (Columbus, Ohio, US) see page 657, abstract no. 102602m; & JP-A-7727780 (UEDA, TORU et al.) 2 March 1977
- Journal of Medicinal Chemistry, vol. 20, no. 1, 1977 (Columbus, Ohio, US) D.H. Hollenberg et al.: "Nucleosides. 102. Synthesis of some 3'-deoxy-3'- substituted arabinofuransosylpyrimidine nucleosides", pages 113-116 see compound 5a,b
- Journal of Organic Chemistry, vol. 39, no. 11, 1974 (Columbus, Ohio, US) M.J. Robins et al.: "Nucleic acid related compounds. 11. Adenosine 2',3'-ribo-epoxide. Synthesis, intramolecular degradation, and transformation into 3'-substituted xylofuranosyl nucleosides and the lyxo-epoxide" pages 1564-1570, see compound 10
- Chemical Abstracts, vol. 72, 1970 (Columbus, Ohio, US) see page 352, abstract no. 3725n; & JP-A-6917910 (DAIICHI SEIYAKU CO. LTD) 06th August 1969
- Journal of Organic Chemistry, vol. 44, no. 8, 1979 (Columbus, Ohio, US) M.J. Robins et al.: "Nucleic acid related compounds.30. Transformations of adenosine to the first 2',3'-aziridine-fused nucleosides, 9-(2,3-epimino-2,3-dideoxy-beta-D-ribofuranosyl) adenine and 9-(2,3-dideoxy-beta-D-lyxofuranosyl)adenine" pages 1317-1322, see compound 1 and 7
- The Journal of Biological Chemistry, vol. 262, no. 5, 15 February 1987 The American Society of Biological Chemists, Inc. (US) Y.C. Cheng et al.: "Human immuno-deficiency virus reverse stranscriptase", pages 2187-2189

## Description

This invention relates to methods for the therapy and prophylaxis of infections, in particular to infections by retroviruses such as HIV. More specifically, this invention relates to the use and preparation of nucleoside analogues to treat such infections.

Several deoxyribonucleoside analogues are known to be potent inhibitors of retroviral reverse transcriptase (RT), in particular the RT of human immunodeficiency virus (HIV), the virus known to be the causative agent of acquired immunodeficiency syndrome (AIDS)¹. These analogues [e.g., 3'-azido-3'-deoxythymidine (AZT) and 2',3'dideoxycytidine (ddC)] are converted in vivo by cellular kinases to the active 5' triphosphates. The triphosphates are the active agents in inhibiting RT. These triphosphates are recognized by RT as substrates and are thought to be incorporated into DNA. Since they lack a 3'-hydroxyl for elongation of the DNA chain, and since RT has no 3'-5' proofreading ability, DNA synthesis is irreversibly halted. Analogues such as AZT or ddC have properties which make them valuable as AIDS therapeutic agents; that is, the corresponding triphosphates are much better substrates for RT than they are for the cellular DNA polymerase (Pol α). This fact means that these analogues can selectively inhibit RT without halting host DNA synthesis¹.

The rational design of nucleoside analogue inhibitors of RT is limited in several regards; the major limitations are due to the fact that the exact molecular mechanisms of the substrate recognition and catalysis by cellular kinases, RT and Pol α are not known. The reports which have led to the use of AZT and ddC as AIDS therapies followed the synthesis of these compounds by more than a decade¹. These compounds were taken "off the shelf" and applied as AIDS therapies. Thus the nucleoside analogues which are proposed or in use for therapy or prophylaxis of AIDS infection were not designed for that purpose. They exert side effects, e.g. bone marrow toxicity, and are not sufficiently effective to be considered a therapeutic cure. In addition, AZT in particular has been reported to be extremely costly because of its complex synthetic route. Finally, known nucleoside analogues for treatment of retroviral infections act by inducing DNA chain termination, but other mechanisms of action for retroviral RT inhibition or inactivation have not been explored.

Accordingly, it is an object of this invention to reduce or eliminate the toxicity and side-effects of nucleoside analogues at therapeutic dosages.

It is another object to improve the efficacy of nucleoside analogues in the treatment or prophylaxis of retroviral infections.

It is an additional object to provide nucleoside analogues which are less expensive to synthesize than those which are currently in use for the treatment of HIV.

It is a still further object to provide reactive nucleoside analogues which are capable of functioning as suicide substrates for RT.

The following prior art is acknowledged:
(A) references which disclose 2'-3' lyxo-thiirane uracils:
   (1) Chem. Abstracts 87:102602m (1977) (Ueda et al)
   (2) Chem. Abstracts 87:68571p (1977) (Ueda et al).
(B) references which disclose 2'-3' lyxo-oxirane nucleosides:
   (1) Robins, M. et al., J. Organic Chem. 39(11):1564-1569 (1974)
   (2) Hollenberg et al., J. Med. Chem. 20(1):113-116 (1977)
   (3) Hirata et al., Chem. Abstracts 72:3725n (1970)
(C) Robins, M. et al., J. Organic. Chem. 44(8):1317-1322 (1979): this discloses 2'-3' lyxo-aziridinyl nucleosides.
(D) Cheng et al., J. Biol. Chem. 262(5):2187-2189 (1987): this discloses methods of testing nucleoside analogues as potential anti-viral agents.

These and other objects will be apparent to the ordinary artisan from the specification as a whole.

According to the invention there is provided a compound for therapeutic use selected from compounds of the following formulae: 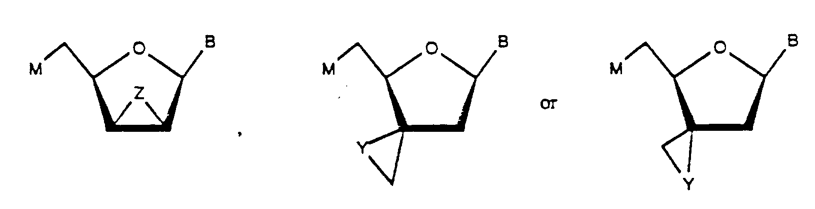
Z is S or NR;
Y is O, NH or NR;
R is acyl;
B is a purine or pyrimidine base or an analogue of such base, other than uracil itself, which is capable of base pairing with adenine, thymine, guanine, cytosine or uracil in a polynucleotide; and
M is hydroxyl or an ester; and their pharmaceutically acceptable salts.

These compounds are useful for anti-infective therapy, including the treatment of retroviral infections. They are formulated into pharmaceutically effective dosage forms for administration to patients, particularly patients infected with HIV.

### Detailed Description of the Invention

In general, and with the exceptions noted above, "B" is any purine or pyrimidine base, or analogue thereof, other than uracil, which is capable of base pairing with polynucleotide cytosine, adenine, thymine, uridine or guanine bases. B is linked to the sugar 1' site through the 9- position of purines or 1- position of pyrimidines. Preferably, base B is cytosinyl or thyminyl, although other bases are suitable. For example, B is selected from among thyminyl, cytosinyl, N⁴- substituted cytosinyl, 0⁴-substituted uridinyl and 5- substituted thyminyl. 0⁴-uridinyl substituents include methyl and -OH, and 5-uridinyl substituents include replacement of the 5 hydrogen with halogen, e.g. bromine. Analogues of the purine bases adenine or guanine include 2,6-diaminopurine, 6-methylthiopurine, 6-methoxypurine, xanthosine, hypoxanthine, purine and 2-amino purine. The nature of the base is not critical so long as it is able to confer on the nucleoside analogue of which it is a part the ability to base pair with a polynucleotide, preferably RNA, under the aegis of RT. Base B of the nucleoside analogues herein will be selected on the basis of being incorporated into DNA by RT or being reactive with RT to a greater degree than with mammalian Pol-α, as is readily determined by in vivo or in vitro screening.

The pentofuranosyl moieties fall within two classes: the beta (or upper) oriented episulfides and aziridines, and the 3' spiro epoxides and aziridines.

The aziridines are expected to be less stable in vivo than the epoxides and episulfide but offer promise as "suicide" substrates for RT. In this scenario, and without limiting the invention to any particular theory of action, nucleophilic attack by an amino acid side chain of RT will result in acylation of the enzyme, in turn inhibiting or inactivating the enzyme. The acyl groups "R" preferably are acetyl or formyl since these offer the least steric bulk. However, other acyl substituents also are believed to be usable.

M is preferably hydroxyl. However, esters are also suitable, particularly those which are hydrolyzed in vivo to yield 5' hydroxyl. Such esters are useful in sustained release formulations wherein endogenous esterolytic enzymes gradually release the 5' hydroxyl species. Esters include carboxylic acid esters in which the non-carbonyl moiety of the ester grouping is selected from straight or branched chain alkyl (about 1-18 carbon atoms, preferably 1-4), alkoxyalkyl (e.g. methoxymethyl), aralkyl (e.g. benzyl), arloxyalkyl (e.g. phenoxymethyl), aryl (e.g. phenyl optionally substituted by halogen, C₁₋₄ alkyl or C₁₋₄ alkoxy); and sulphonate esters such as alkyl- or aralkylsulphonyl (e.g. methanesulphonyl). Mono-, di-, or triphosphate esters are not preferred because they are not believed to cross cell membranes as readily as compounds having more hydrophobic 5' substituents, although it will be appreciated that cellular protein kinases will triphosphorylate the 5' hydroxyl, thereby creating the chain terminating species.

Any reference to any of the compounds herein also includes the pharmaceutically acceptable salts of such compounds. Examples of pharmaceutically acceptable salts include those of alkaline earths (e.g. sodium or magnesium), ammonium or NX₄⁺ (wherein X is C₁₋₄ alkyl). Other pharmaceutically acceptable salts include organic carboxylic acids such as acetic, lactic, tartaric, malic, isethionic, lactobionic and succinic acids; organic sulfonic acids such as methanesulfonic, ethanesulfonic, benzenesulfonic and p-tolunesulfonic acids and inorganic acids such as hydrochloric, sulfuric, phosphoric and sulfamic acids. Physiologically acceptable salts of a compound having a hydroxy group include the anion of said compound in combination with a suitable cation such as Na⁺, NH₄⁺, and NX₄⁺ (wherein X is a C₁₋₄ alkyl group).

Examples of compounds falling within the scope herein have the structures 1-3, and 5-8: 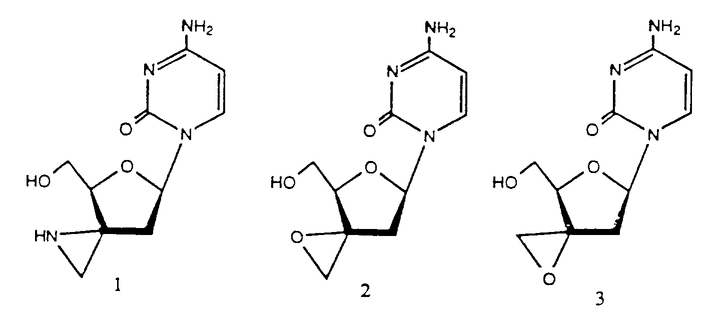 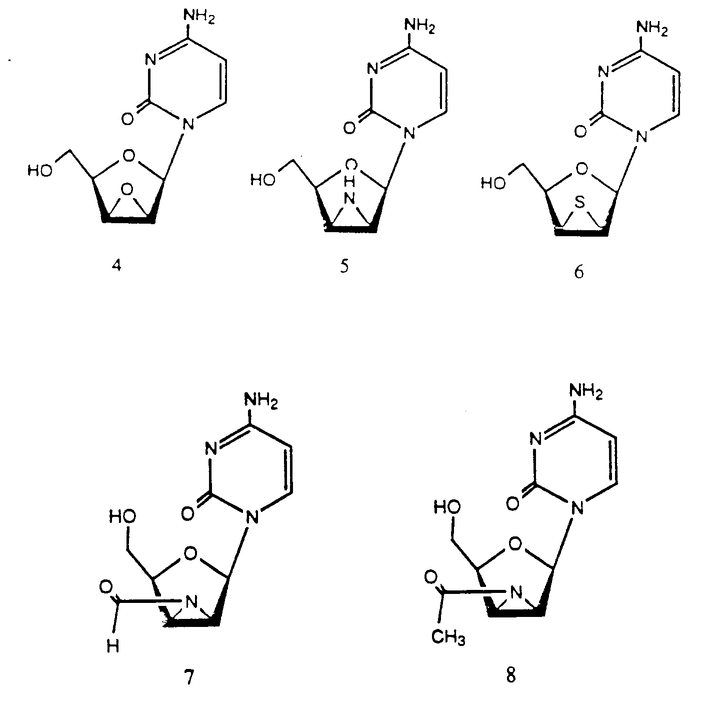
The compounds of this invention are prepared by methods known in the art or modifications thereof as will be apparent to the ordinary artisan. Compound 4 is a known compound and is prepared by the method of Hollenberg et al.⁵ Compound 5 may be prepared by methods available to the art. However, the action of lithium azide on 5'-dimethoxytrityl-3'-mesyluridine followed by removal of the trityl group and treatment of the resulting trans azido alcohol with triphenylphosphine failed to give 5. Compound 5 is converted into 7 or 8 by treatment with the appropriate acid anhydride. Compounds 1 to 3 (designated XI, VI and IX respectively) are made using the following procedure.

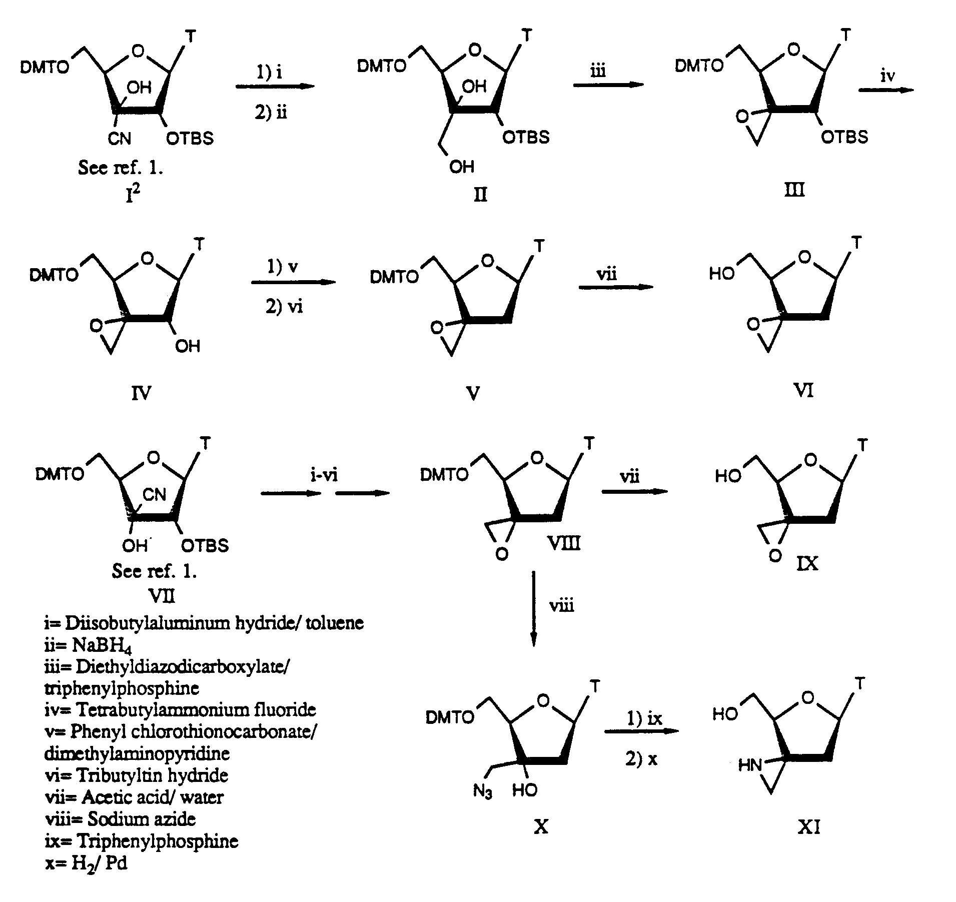

### References;

1) A. Calvo-Mateo, M. Camarasa, A. Diaz-Ortiz and F.G. De las Heras, **Tetrahedron Lett**., 29, 941-944, 1988.
2) T.R. Webb, H. Mitsuya, and S. Broder, **J. Med. Chem**., in press 1988.

The synthesis of 6 may be accomplished by treating 5'-dimethoxytrityl-2',3'-dimesyl-uridine with Na₂S or sodium thioacetate followed by mild base treatment, followed by conversion of the uracil base to a cytosine base by known methods. Alternatively the episulfide can be prepared by known methods, see "Advanced Organic Chemistry", Jerry March, Wiley Interscience, New York (1985) or by modification of the method set forth in Japanese patent application 103,509 (filed 8/28/75) wherein the substituents at the 2' and 3' position of the starting material have the opposite orientation from that which is shown in the application, i.e. 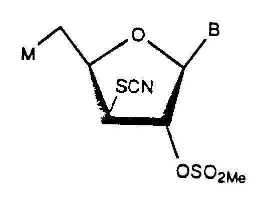
The synthesis of purine-β-2',3'epoxynucleosides can be accomplished according to the procedure of Robins, M.J. et al.⁴

Esters at M include monophosphates; diphosphate; triphosphate; acetate; 3-methyl-butyrate; octanoate; palmitate; 3-chloro benzoate; benzoate; 4-methyl benzoate; hydrogen succinate; pivalate; and mesylate.

The compounds according to the invention are administered for therapy of infectious agents by any suitable route including oral, rectal, nasal, topical (including buccal and sublingual), vaginal and parenteral (including subcutaneous, intramuscular, intravenous and intradermal). It will be appreciated that the preferred route and dosage will vary with the condition and age of the recipient, the infection involved, side effects such as anemia, the clinical condition of the patient, the identity of the infectious agent and other parameters which the skilled clinician typically encounters.

The compounds according to the invention are active against viruses, in particular retroviruses such as lymphotropic viruses (HTLV), especially HTLV-I, HTLV-II and HIV. The invention accordingly provides the compounds according to the invention for use in the treatment or prophylaxis of the above infections.

In general a suitable dose will be in the range of 1.0 to 50 mg per kilogram body weight of the recipient per day, preferably in the range of 3 to 30 mg per kilogram body weight per day and most preferably in the range of 5 to 15 mg per kilogram body weight per day. The desired dose is preferably presented as two, three, four, five, six or more sub-doses administered at appropriate intervals throughout the day, or is administered by continuous i.v. pump. Sub-doses may be administered in unit dosage forms, for example, containing 2 to 25 mg, preferably 3 to 20 mg, and most preferably 4 to 10 mg of active ingredient per unit dosage form. The active ingredient should be administered to achieve peak plasma concentrations of the active compound of from about 1 to about 100 »M, preferably about 3 to 50 »M, most preferably about 5 to about 15 »M. The therapeutic amounts of compounds having Z = S or NR, or Y=O or NR are expected to be less than required for the remaining compounds, and lower than the ranges set forth in this paragraph.

While it is possible for the active ingredient to be administered alone, it is preferable to present it as a pharmaceutical formulation. The formulations of the present invention comprise at least one active ingredient, as above defined, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. providone, gelatin, hydroxypropylmethyl cellulose), lubricant, inert diluent, preservative, disintegrant (e.g. sodium starch glycolate, cross-linked povidone, cross-linked sodium carboxymethyl cellulose) surface-active or dispersing agent. Molded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous where, B is a purine and/or Z is NH or S since such compounds are susceptible to acid hydrolysis.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or salicylate.

Formulations suitable for vaginal administration may be presented as pessaries, tampons, creams, gels, pasts, foams or spray formulations containing in addition to the active ingredient such carriers as are known in the art to be appropriate.

Formulations suitable for parenteral administration include aqueous and non-aqueous isotonic sterile injection solutions which may contain antioxidants, buffers, bacteriostats and solutes which render the formulation isotonic with the blood of the intended recipient; and aqueous non-aqueous sterile suspensions which may include suspending agents and thickening agents. The formulations may be presented in unit-dose or multi-dose sealed containers, for example, minibags, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

The compounds according to the invention may also be presented for use in the form of veterinary formulations, which may be prepared, for example, by methods that are conventional in the art. Examples of such veterinary formulations include those adapted for:
(a) oral administration, external application, for example drenches (e.g. aqueous or non-aqueous solutions or suspensions); tablets or boluses; powders, granules or pellets for admixture with feed stuffs; pastes for application to the tongue;
(b) parenteral administration for example by subcutaneous, intramuscular or intravenous injection, e.g. as a sterile solution or suspension; (when appropriate) by intramammary injection where a suspension or solution is introduced into the udder via the teat;
(c) topical application, e.g. as a cream, ointment or spray applied to the skin; or
(d) intravaginally, e.g. as a pessary, cream or foam.

The administered ingredients may also be used in therapy in conjunction with other medicaments such as antibiotics, 9-[[2-hydroxy-1-(hydroxymethyl)ethoxy]methyl]guanine, 2-amino-9-(2-hydroxyethoxymethyl)purine, interferon, e.g., γ or α interferon, tumor necrosis factor, interleukin II, AZT, ddC and phosphonoformate, as is appropriate. In one treatment embodiment, retroviral replication cycles are induced by immunostimulants such as bacterial peptides or lymphokines, during which period the compounds of the invention are administered to the subject, optionally together with tumor necrosis factor. Thereafter, an intermediate stage without immunostimulation and, optionally, coupled with compound administration is permitted to pass, after which the compound treatment cycle is repeated.

### EXAMPLE 1

Note: this example discloses a technique for demonstrating the therapeutic effect of a compound. However the compound used is not according to the invention.

Compound 4, AZT and ddC were diluted into PBS and used to produce solutions of cell culture medium in which the concentrations of 4 were 1, 10 and 100 »M 1, 5 and 50 »M (AZT) and 0.1 and 10 »M (ddC). The effect of these compounds on the growth of HIV infected ATH8 cells (2 x 10⁵ cells per tube; 2000 virus particles/cell) was determined by the method of Broder et al.¹ A compound control contained the candidate compound but the cells were not virally infected. A treatment control contained no compound nor were the cells infected with virus. Viable cells were counted in each tube after six days incubation. Percent protection was equal to 100 x (the number of cells surviving the viral infection divided by the number of cells in the compound control). The percent toxicity was equal to 100 x (the number of surviving cells in the compound control divided by the number of cells in the treatment control).

The results are shown in the table below.

**TABLE**

| Candidate | Concentration (»M) | Protection (%) | Toxicity (%) |
|---|---|---|---|
| AZT | 1, 5, 50 | 78, 98, 47 | 3, 11, 50 |
| ddC | 0.1, 1, 10 | 12, 119, 96 | 0, 0, 4 |
| 4 | 1, 10, 100 | 20, 100, 50 | 0, 30, 50 |

This demonstrates that compound 4 confers substantial protection against HIV infection and is substantially nontoxic at concentrations below about 5 »M. The adenyl derivative of compound 4 was comparatively weakly active in conferring protection.

### REFERENCES

1. Broder, S. "AIDS: Modern Concepts and Therapeutic Challenges"; Marcel Dekker: New York and Basel, 1987.
2. A. Calvo-Mateo, M. Camarasa, A. Diaz-Ortiz and F.G. De las Heras, Tetrahedron Lett. 29:941-944 (1988).
3. Kim, C. Marquez, V.E., Broder, S., Mitsuya, H. and Driscoll, J.S. "J. Med. Chem." 30:862-866 (1987).
4. Robins, M.J.; Hansske, F.; Low, N.H.; Park, J.I.; "Tet. Lett." 25:367 (1984).
5. Hollenberg , D.; Watanabe, K.; Fox, J. "J. Med. Chem. 41:2042 (1976).

## Claims

1. A compound for therapeutic use selected from compounds having the following formulae: 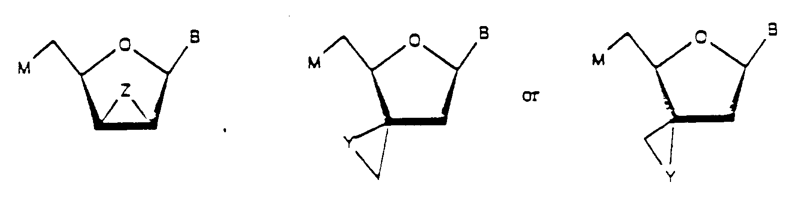 wherein
Z is S or NR;
Y is O, NH or NR;
R is acyl;
B is purine or pyrimidine base or an analog thereof, other than uracil itself, which is capable of base pairing with adenine, thymine, guanine, cytosine or uracil in a polynucleotide; and
M is hydroxyl or an ester;
and their pharmaceutically acceptable salts.

2. The compound of claim 1 which is

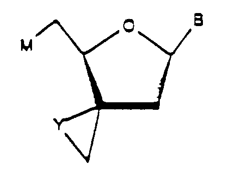

3. The compound of claim 2 wherein B is cytosine or adenine and Y is O.

4. The compound of claim 2 whrein Y is NH, M is hydroxyl and B is cytosine.

5. The compound of claim 1 wherein Z is S, M is hydroxyl and B is cytosine, thymine, adenine or guanine.

6. The compound of claim 1 wherein Z is NR, M is hydroxyl and B is other than cytosine, thymine, adenine, or guanine.

7. The compound of claim 6 wherein R is hydrogen.

8. The compound of claim 6 or 7 wherein B is selected from N⁴-substituted cytosine, O⁴-substituted uracil, 5-substituted uracil, 2,6-diaminopurine, 6-methylpurine, 6-methoxypurine, xanthosine, hypoxanthine, 2-amino purine and purine.

9. The compound of claim 2 wherein Y is O.

10. The compound

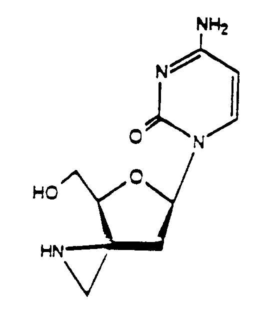

11. The compound

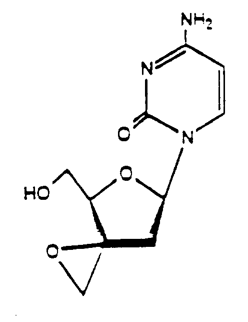

12. The compound

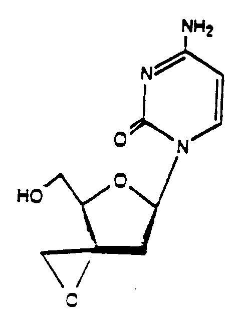

13. The compound

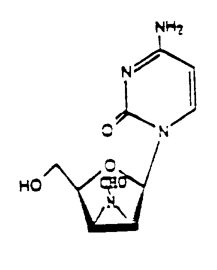

14. The compound

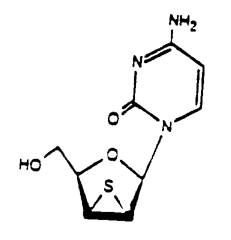

15. A composition for therapeutic use comprising a compound according to any preceding claim and an interferon and a tumor necrosis factor for simultaneous or sequential administration.

16. A therapeutic composition comprising a plurality of compounds according to any of claims 1-14.

17. A pharmaceutical preparation comprising a physiologically innocuous carrier and a compound selected from compounds having the following formulae: 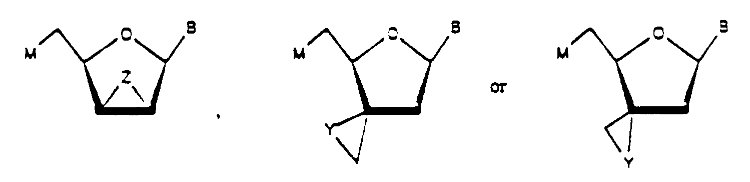 wherein
Z is S or NR;
Y is O, NH or NR:
R is acyl;
B is purine or pyrimidine base or an analog thereof, other than uracil, which is capable of base pairing with adenine, thymine, guanine, cytosine or uracil in a polynucleotide; and
M is hydroxyl or an ester;
and their pharmaceutically acceptable salts.

18. The preparation of claim 17 which is sterile.

19. The preparation of claim 17 or 18 which is packaged into a unitary dosage form.

20. The preparation of claim 19 wherein the unitary dosage form is a tablet or capsule.

21. The preparation of claim 17 wherein the carrier is substantially isotonic.

22. The preparation of claim 17 wherein the carrier is a sustained release polymer.

23. Use of a compound of any of claims 1-14 for the manufacture of a medicament for therapeutic treatment of a subject infected with HIV.

24. Use of a compound according to any of claims 1-14 for the manufacture of a medicament for a therapeutic treatment comprising stimulating the immune system of the patient while administering the compound, followed by withdrawing the immune stimulus.

25. Use according to claim 24 for a method which is repeated through a plurality of cycles.

26. Use according to claim 23, 24 or 25 for a method wherein the dose is sufficient to produce a plasma concentration of the compound in the subject ranging about from 1 to 50»M.

27. Use according to claim 23, 24 or 25 for a method wherein the dose is sufficient to produce a plasma concentration of about from 1 to 15»M.

## Patentansprüche

1. Verbindung zur therapeutischen Verwendung, ausgewählt aus Verbindungen mit den folgenden Formeln: 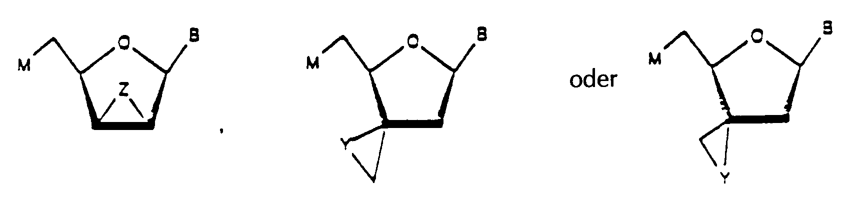 worin
Z S oder NR ist;
Y O, NH oder NR ist;
R Acyl ist;
B eine Purin- oder Pyrimidinbase oder ein Analog davon, mit Ausnahme von Uracil selbst, ist, die bzw. das zur Basenpaarung mit Adenin, Thymin, Guanin, Cytosin oder Uracil in einem Polynukleotid fähig ist; und
M Hydroxyl oder ein Ester ist; und ihre pharmazeutisch annehmbaren Salze.

2. Verbindung nach Anspruch 1, nämlich:

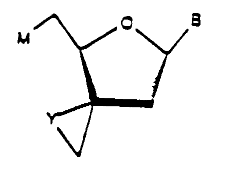

3. Verbindung nach Anspruch 2, worin B Cytosin oder Adenin und Y O ist.

4. Verbindung nach Anspruche 2, worin Y NH ist, M Hydroxyl ist und B Cytosin ist.

5. Verbindung nach Anspruch 1, worin Z S ist, M Hydroxyl ist und B Cytosin, Thymin, Adenin oder Guanin ist.

6. Verbindung nach Anspruch 1, worin Z NR ist, M Hydroxyl ist und B etwas anderes als Cytosin, Thymin, Adenin oder Guanin ist.

7. Verbindung nach Anspruch 6, worin R Wasserstoff ist.

8. Verbindung nach Anspruch 6 oder 7, worin B aus N⁴-substituiertem Cytosin, O⁴-substituiertem Uracil, 5-substituiertem Uracil, 2,6-Diaminopurin, 6-Methylpurin, 6-Methoxypurin, Xanthosin, Hypoxanthin, 2-Aminopurin und Purin ausgewählt ist.

9. Verbindung nach Anspruch 2, worin Y O ist.

10. Verbindung, nämlich

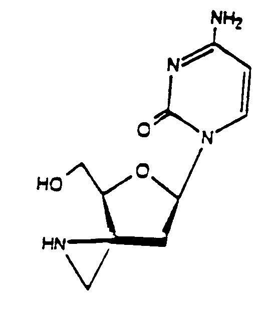

11. Verbindung, nämlich

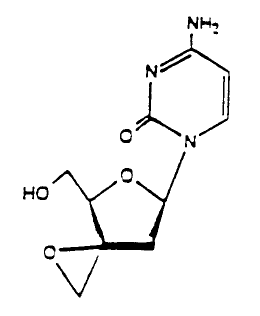

12. Verbinundung, nämlich

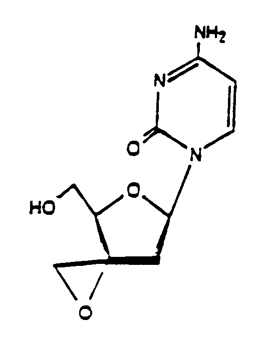

13. Verbindung, nämlich

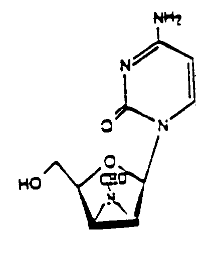

14. Verbindung, nämlich

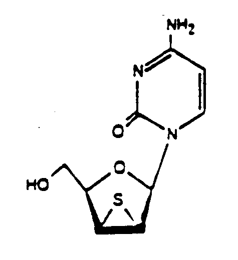

15. Zusammensetzung zur therapeutischen Verwendung, umfassend eine Verbindung nach einem der vorhergehenden Ansprüche und ein Interferon und einen Tumornekrosefaktor zur gleichzeitigen oder aufeinanderfolgenden Verabreichung.

16. Therapeutische Zusammensetzung umfassend eine Vielzahl an Verbindungen nach einem der Ansprüche 1-14.

17. Pharmazeutisches Präparat umfassend einen physiologisch harmlosen Träger und eine Verbindung, ausgewählt aus den Verbindungen mit den folgenden Formeln: 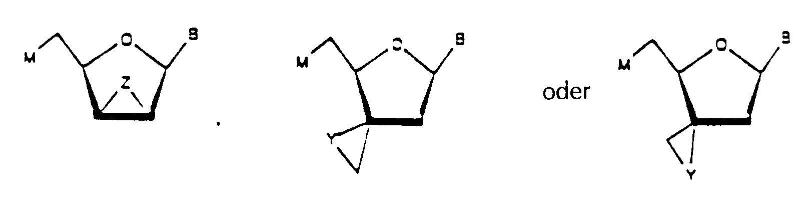 worin
Z S oder NR ist;
Y O, NH oder NR ist;
R Acyl ist;
B eine Purin- oder Pyrimidinbase oder ein Analog davon, mit Ausnahme von Uracil selbst, ist, die bzw. das zur Basenpaarung mit Adenin, Thymin, Guanin, Cytosin oder Uracil in einem Polynukleotid fähig ist; und
M Hydroxyl oder ein Ester ist;
und ihren pharmazeutisch annehmbaren Salzen.

18. Präparat nach Anspruch 17, das steril ist.

19. Präparat nach Anspruch 17 oder 18, das in eine Einheitsdosierungsform verpackt ist.

20. Präparat nach Anspruch 19, worin die Einheitsdosierungsform eine Tablette oder Kapsel ist.

21. Präparat nach Anspruch 17, worin der Träger im wesentlichen isotonisch ist.

22. Präparat nach Anspruch 17, worin der Träger ein Polymer mit Langzeit-Freisetzung ist.

23. Verwendung einer Verbindung nach einem der Ansprüche 1-14 zur Herstellung eines Medikaments zur therapeutischen Behandlung eines mit HIV infizierten Patienten.

24. Verwendung einer Verbindung nach einem der Ansprüche 1-14 zur Herstellung eines Medikaments zur therapeutischen Behandlung, umfassend das Stimulieren des Immunsystem des Patienten während des Verabreichens der Verbindung, und das anschließende Entziehen des Immunstimulus.

25. Verwendung nach Anspruch 24 für ein Verfahren, das in einer Vielzahl an Zyklen wiederholt wird.

26. Verwendung nach Anspruch 23, 24 oder 25 für ein Verfahren, worin die Dosis ausreichend ist, eine Plasmakonzentration der Verbindung im Patienten zu erzeugen, die etwa von 1 bis 50 »M reicht.

27. Verwendung nach Anspruch 23, 24 oder 25 für ein Verfahren, worin die Dosis ausreicht, eine Plasmakonzentration etwa von 1 bis 15 »M zu erzeugen.

## Revendications

1. Composé destiné à une utilisation thérapeutique, choisi entre des composés répondant à la formule suivante : 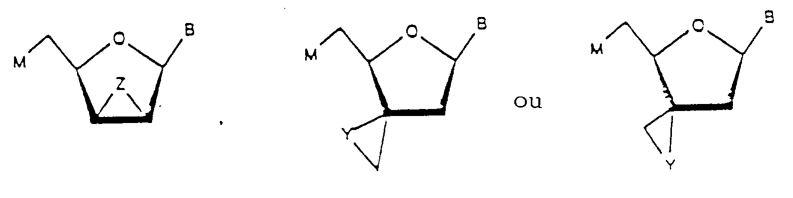 dans laquelle
Z représente S ou un groupe NR ;
Y représente O, un groupe NH ou NR ;
R représente un groupe acyle ;
B représente une base purique ou pyrimidique ou un de ses analogues, autre que l'uracile proprement dit, qui est capable d'un appariement de bases avec l'adénine, la thymine, la guanine, la cytosine ou l'uracile dans un polynucléotide ; et
M représente un groupe hydroxyle ou un ester ; et ses sels pharmaceutiquement acceptables.

2. Composé suivant la revendication 1, qui répond à la formule

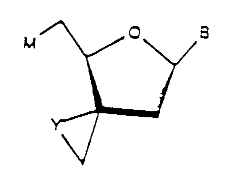

3. Composé suivant la revendication 2, dans lequel a représente la cytosine ou l'adénine et Y représente O.

4. Composé suivant la revendication 2, dans lequel Y représente un groupe NH, M représente un groupe hydroxyle et B représente la cytosine.

5. Composé suivant la revendication 1, dans lequel Z représente S, M représente un groupe hydroxyle et B représente la cytosine, la thymine, l'adénine ou la guanine.

6. Composé suivant la revendication 1, dans lequel Z représente un groupe NR, M représente un groupe hydroxyle et B est autre que la cytosine, la thymine, l'adénine ou la guanine.

7. Composé suivant la revendication 6, dans lequel R représente l'hydrogène.

8. Composé suivant la revendication 6 ou 7, dans lequel B est choisi entre une cytosine N⁴-substituée, un uracile O⁴-substitué, un uracile 5-substitué, la 2,6-diaminopurine, la 6-méthylpurine, la 6-méthoxypurine, la xanthosine, l'hypoxanthine, la 2-aminopurine et la purine.

9. Composé suivant la revendication 2, dans lequel Y représente O.

10. Composé de formule

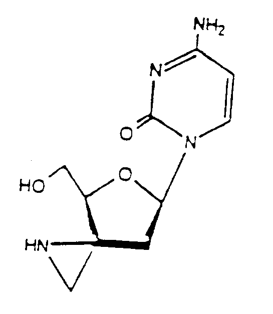

11. Composé de formule

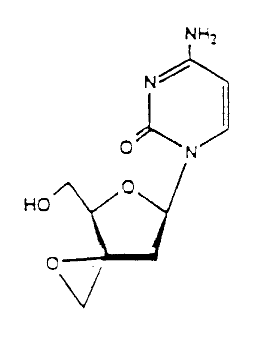

12. Composé de formule

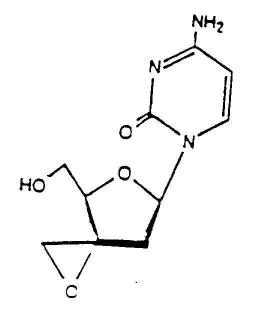

13. Composé de formule

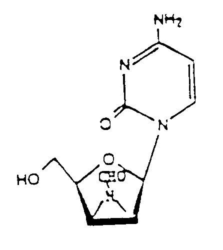

14. Composé de formule

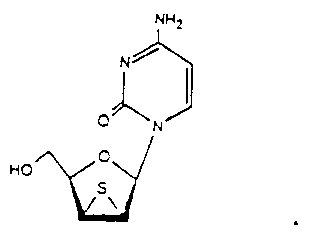

15. Composition destinée à une utilisation thérapeutique, comprenant un composé suivant l'une quelconque des revendications précédentes et un interféron ainsi qu'un facteur de nécrose tumorale pour une administration simultanée ou séquentielle.

16. Composition thérapeutique comprenant une pluralité de composés suivant l'une quelconque des revendications 1 à 14.

17. Préparation pharmaceutique comprenant un support doué physiologiquement d'innocuité et un composé choisi entre des composés répondant à la formule suivante : 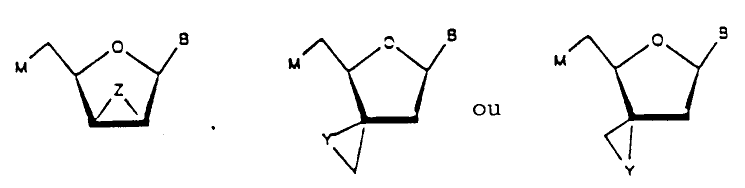 dans laquelle
Z représente S ou un groupe NR ;
Y représente O, un groupe NH ou NR ;
R représente un groupe acyle ;
B représente une base purique ou pyrimidique ou un de ses analogues, autre que l'uracile, qui est capable d'un appariement de bases avec l'adénine, la thymine, la guanine, la cytosine ou l'uracile dans un polynucléotide ; et
M représente un groupe hydroxyle ou un ester ; ou un de ses sels pharmaceutiquement acceptables.

18. Préparation suivant la revendication 17, qui est stérile.

19. Préparation suivant la revendication 17 ou 18, qui est conditionnée sous une forme posologique unitaire.

20. Préparation suivant la revendication 19, dans laquelle la forme posologique unitaire est un comprimé ou une capsule.

21. Préparation suivant la revendication 17, dans laquelle le véhicule est pratiquement isotonique.

22. Préparation suivant la revendication 17, dans laquelle le véhicule est un polymère à libération prolongée.

23. Utilisation d'un composé suivant l'une quelconque des revendications 1 à 14 pour la production d'un médicament destiné au traitement thérapeutique d'un sujet infecté par le HIV.

24. Utilisation d'un composé suivant l'une quelconque des revendications 1 à 14 pour la production d'un médicament destiné à un traitement thérapeutique comprenant la stimulation du système immunitaire du patient avec administration simultanée du composé, puis la suppression du stimulus immunitaire.

25. Utilisation suivant la revendication 24 pour un procédé qui est répété par mise en oeuvre d'une pluralité de cycles.

26. Utilisation suivant la revendication 23, 24 ou 25 pour un procédé dans lequel la dose est suffisante pour produire chez le sujet une concentration plasmatique du composé allant d'environ 1 à 50 »M.

27. Utilisation suivant la revendication 23, 24 ou 25 pour un procédé dans lequel la dose est suffisante pour produire une concentration plasmatique d'environ 1 à 15 »M.
